# EUROPEAN PATENT SPECIFICATION

(11) **EP 1 281 398 B1**
(45) Date of publication and mention of the grant of the patent: **30.07.2008**
(21) Application number: 01929996.5
(22) Date of filing: 09.05.2001
(51) Int. Cl.: A61K 31/05, A61K 31/122, A61K 9/06, A61K 9/107, A61P 37/08, A61P 17/00, A61P 17/02, A61P 17/04, A61P 17/06, A61P 17/08, A61K 8/00, A61Q 17/00

(54) **DERMAL COMPOSITIONS CONTAINING COENZYME Q AS THE ACTIVE INGREDIENT**
DERMALE ZUSAMMENSTELLUNGEN DIE ALS WIRKSTOFF COENZYM Q ENTHALTEN
COMPOSITIONS DERMIQUES AYANT LA COENZYME Q COMME PRINCIPE ACTIF

(30) Priority: 09.05.2000 JP 2000135568
(43) Date of publication of application: 05.02.2003
(73) Proprietor: KANEKA CORPORATION, Osaka-shi, Osaka 530-8288 (JP)
(72) Inventor: FUJII, Kenji, Kobe-shi, Hyogo 651-1202 (JP); KAWABE, Taizo, Takasago-shi, Hyogo 676-0082 (JP); HOSOE, Kazunori, Takasago-shi, Hyogo 676-0025 (JP); HIDAKA, Takayoshi, Kobe-shi, Hyogo 655-0006 (JP)
(74) Representative: Gille Hrabal Struck Neidlein Prop Roos
(86) International application number: PCT/JP2001/003863
(87) International publication number: WO 2001/085156

(56) References cited:
- EP-A- 0 261 362
- WO-A-98/35658
- WO-A-98/35660
- WO-A1-95/05852
- DD-A1- 273 002
- JP-A- 2 059 519
- JP-A- 58 180 410
- JP-A- 59 013 719
- JP-A- 61 027 914
- US-A- 6 048 886

## Description

### TECHNICAL FIELD

The present invention relates to a composition for dermal application which contains a coenzyme Q as an active ingredient, in particular to a composition for the treatment of skin diseases, a cosmetic composition, a skin health care composition and a bath salt composition.

### BACKGROUND ART

Coenzymes Q are physiologically essential factors distributed widely in living organisms, from bacteria to mammals, and occur as constituents of the mitochondrial electron transport system in cells of the living organism. Coenzymes Q function as carrier components in the electron transport system by repeating oxidation and reduction in vivo, and reduced coenzymes Q are also known as antioxidants. In many animals inclusive of human beings, or in fish and birds, coenzyme Q₁₀, which is a coenzyme Q whose side chain comprises 10 repetitions of a unit, is predominant. Further, it is known that about 40 to 90% of this coenzyme Q₁₀ occurs in reduced form in living organisms.

As for the practical uses of coenzymes Q, oxidized coenzyme Q₁₀, for instance, has been used as a drug for congestive heart failure and, in other fields than the pharmaceutical field, it has been used widely as a nutrient or nutritional supplement, like vitamins. However, reduced coenzyme Q₁₀ has not yet been put to practical use.

In Japanese Kohyo Publication Hei-09-501925, there is disclosed a dermal preparation containing oxidized coenzyme Q₁₀ (ubiquinone) or reduced coenzyme R₁₀ (ubiquinol) as a coenzyme Q₁₀. In this document, however, it is disclosed only as one of a large number of examples of the active ingredient. As regards ubiquinol, in particular, no example is given for the actual use thereof. It is described that such coenzyme Q₁₀-containing dermal preparations are effective against atopic dermatitis. However, the name of that disease, too, appears only as an example of a large number of skin diseases. There is no relevant example, hence the actual effect is unknown.

In Japanese Kokai Publication Hei-10-109933, the inventors of the present invention disclosed that the combined use of reduced coenzyme Q₁₀ and oxidized coenzyme Q₁₀ results in an improvement in oral absorbability as compared with the single use of oxidized coenzyme Q₁₀. However, the effect of reduced coenzymes Q on absorbability upon administration via other routes than the oral or its efficacy in atopic dermatitis was quite unknown.

It is a problem that skin diseases exert great influences on the life of patients not only physically but also mentally. In particular, the number of patients suffering from the intractable skin disease atopic dermatitis, among others, is tremendous and, further, the number of adult patients with atopic dermatitis has been increasing in recent years, causing serious problems in their leading a social life.

Steroids are generally known as therapeutic agents for atopic dermatitis. However, their use is restricted in not a few instances because of their significant side effects and the possibility of their causing the rebound phenomenon. Therefore, they are not sufficiently effective agents to bring about complete recovery from atopic dermatitis. Furthermore, in cases where steroids are ineffective, there are, in fact, no therapeutic drugs available. It is also a social problem that there are victims of folk medicine.

Tacrolimus, which is an immunosuppressive, has recently been approved as a therapeutic agent for atopic dermatitis. However, its use in children has not yet been approved because of a strong fear of its producing side effects; thus, it cannot be said to be a safe agent.

Under such circumstances, the advent of a therapeutic agent that can be used safely against atopic dermatitis is earnestly demanded.

### SUMMARY OF THE INVENTION

It is an object of the present invention to provide a composition for dermal application which contains coenzyme Q, in particular coenzyme Q₁₀, as an active ingredient, and thus provide a safe and highly effective therapeutic agent for skin diseases, in particular atopic dermatitis.

As a result of investigations made by the present inventors to solve the problems mentioned above, it was found that a combination of reduced and oxidized coenzyme Q₁₀ can produce an excellent therapeutic effect on atopic dermatitis.

The inventors of the present invention prepared a dermal preparation containing reduced coenzyme Q₁₀ and carried out a percutaneous absorption test, whereupon it was found that when a composition containing a certain proportion of reduced coenzyme Q₁₀ as coenzyme Q₁₀ is applied to the skin, a higher level of percutaneous absorption can be attained as compared with a composition containing oxidized coenzyme Q₁₀ alone and the amount of coenzyme Q₁₀ in the skin can be much increased. Furthermore, it was surprisingly found that the content of reduced coenzyme Q₁₀, which is the active principle showing antioxidant activity, in skin can be markedly increased by applying a composition containing reduced coenzyme Q₁₀ as compared with the application of oxidized coenzyme Q₁₀ alone. Heretofore, it has been considered that oxidized coenzyme Q₁₀, when administered, is converted to the reduced form in vivo and thus can show antioxidant activity. However, our studies revealed that the reduction of oxidized coenzyme Q₁₀ in skin proceeds only very slowly and, therefore, the reduced form level is far inferior to that attainable by application of a composition containing reduced coenzyme Q₁₀. By increasing the content in skin of reduced coenzyme Q₁₀, which shows strong antioxidant activity, it becomes possible to expect higher levels of skin care activity as compared with the application of oxidized coenzyme Q₁₀ alone.

Further, the inventors of the present invention evaluated an ointment containing reduced coenzyme Q₁₀ for efficacy in the treatment of atopic dermatitis. As a result, it was found that a reduced coenzyme Q₁₀-containing ointment is by itself highly effective and comparable in therapeutic effect to prednisolone. It was also found that when a reduced coenzyme Q₁₀-containing ointment is used in combination with a steroid or tacrolimus, a more powerful therapeutic effect can be produced.

Furthermore, the inventors of the present invention found that such dermal preparation containing coenzyme Q₁₀ as main active ingredient has a skin restoration promoting activity. This suggests that coenzyme Q₁₀ be effective also against skin diseases, typically decubitus.

Thus, the present invention provides a composition for dermal application which comprises, as an active ingredient, an oxidized coenzyme Q represented by the formula (1): 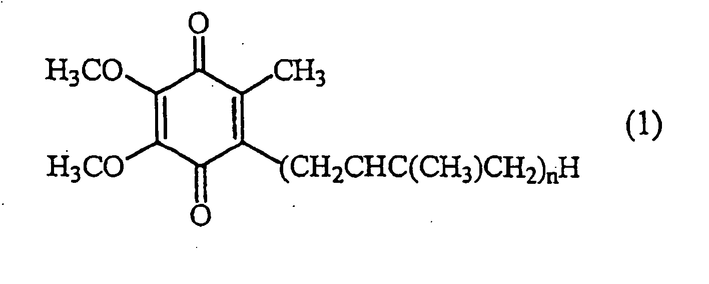 wherein n represents an integer of 1 to 12, and a reduced coenzyme Q represented by the formula (2): 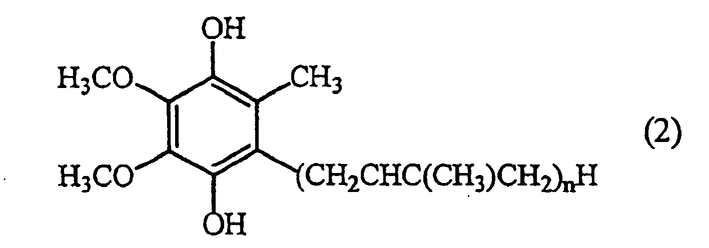 wherein n represents an integer of 1 to 12, the total content of the oxidized coenzyme Q and reduced coenzyme Q being 0.01 to 99% by weight relative to the whole amount of the composition.

The present invention also relates to a therapeutic composition for skin diseases, a cosmetic composition, a skin health care composition and a bath salt composition, each comprising the above composition for dermal application.

In the following, the present invention is described in detail.

### DETAILED DISCLOSURE OF THE INVENTION

The compounds represented by the above formula (1) are oxidized coenzymes Q, while the compounds represented by the above formula (2) are reduced coenzymes Q.

The method of obtaining oxidized coenzymes Q and reduced coenzymes Q is not particularly restricted but the coenzymes Q can be obtained in the conventional manner, for example by synthesis, fermentation, or extraction from natural sources. Or, also employable is a method comprising, for example, subjecting the product obtained in the above manner to chromatography and concentrating the oxidized coenzyme Q fraction or reduced coenzyme Q fraction in an eluate. The oxidized form of coenzyme Q can be obtained by a method known in the art. The reduced from of coenzyme Q may be obtained by adding a conventional reducing agent, such as sodium borohydride or sodium dithionite (sodium hydrosulfite), as necessary, to the above coenzyme Q and reducing the oxidized form of coenzyme Q contained in the above coenzyme Q to the reduced form of coenzyme Q in a conventional manner, followed by concentration by chromatography. It is also possible to obtain the reduced form of coenzyme Q by treating an existing highly pure coenzyme Q with such as a reducing agent as mentioned above.

The method of obtaining the composition of the present invention is not particularly restricted but the composition can be obtained, for example, by dissolving the reduced form of coenzyme Q obtained in the above manner and the oxidized form of coenzyme Q, which is commercially available or obtained by a method known in the art, either in admixture or individually, in an appropriate base. Alternatively, the mixture of reduced coenzyme Q and oxidized coenzyme Q as obtained in the above-mentioned process for coenzyme Q production may be dissolved as such in a base. The base may be selected according to need from among those conventionally used in pharmaceutical preparations, cosmetics and the like within the limits within which the effects of the present invention will not be lessened.

In the composition of the present invention, the total proportion of the oxidized coenzyme Q and reduced coenzyme Q relative to the whole amount of the composition (proportion of the oxidized coenzyme Q relative to the whole composition when the oxidized coenzyme Q alone is contained therein, or proportion of the reduced coenzyme Q relative to the whole composition when the reduced coenzyme Q alone is contained therein) is 0.01 to 99% by weight, preferably 0.1 to 95% by weight, more preferably 0.5 to 50% by weight, still more preferably 1 to 30% by weight.

From the percutaneous absorbability viewpoint, the proportion of the reduced coenzyme Q relative to the total amount of the oxidized coenzyme Q and reduced coenzyme Q is not less than 20% by weight, preferably not less than 40% by weight. Further, the proportion of the reduced coenzyme Q relative to the total amount of the oxidized coenzyme Q and reduced coenzyme Q is preferably not more than 95% by weight.

The oxidized coenzyme Q and reduced coenzyme Q which can be used in the practice of the present invention have a side chain in which, as shown by the above formulas (1) and (2), the number (n in each formula) of repetitions of the repeating unit is 1 to 12. Among them, those in which the number of repetitions of the repeating unit is 10, namely oxidized coenzyme Q₁₀ and reduced coenzyme Q₁₀, are particularly preferred.

The above composition for dermal application, therapeutic composition for skin diseases, cosmetic composition, skin health care composition and bath salt composition may be intended for application to humans or for application to pets, domestic animals and/or birds, in particular dogs and/or cats.

The dosage form of the dermal composition of the present invention is not particularly restricted but includes, among others, cream-like, paste-like, jelly-like, gel-like, emulsion-like or liquid dosage forms prepared by dissolving or dispersing together the above agent(s) in appropriate bases (ointments, liniments, lotions, sprays, etc.), dosage forms prepared by spreading a solution or dispersion of the above agent(s) in a base onto supporting members (poultices etc.), and dosage forms prepared by spreading a solution or dispersion of the above agent(s) in a pressure sensitive adhesive composition onto supporting members (plasters, tapes, etc.).

The dermal composition of the present invention can be used as a therapeutic composition for skin diseases. The skin diseases which can be treated with the composition include, but are not limited to, atopic dermatitis, decubitus, wounds, burns, psoriasis, eruptions, contact dermatitis, seborrheic dermatitis, lichen simplex chronicus Vidal, nummular eczema, housewives' eczema, solar dermatitis, pruritus cutaneus, prurigo Devergie, drug eruption, lichen planus, pityriasis rubra pilaris, pityriasis rosea Gibert, erythema, erythrodermia, wounds, athlete's foot, and skin ulcer, among others.

In using the dermal composition of the present invention as a therapeutic composition for skin diseases, the composition may further contain a substance showing antioxidant activity, for example superoxide dismutase, catalase, glutathione peroxidase, vitamin E, vitamin C, glutathione, glutathione reductase, a polyvalent unsaturated fatty acid or the like. It may also contain a skin activating ingredient, for example collagen, hyaluronic acid, mutin, a ceramide, squalene, squalane or the like, or a percutaneous absorption promoter.

It may further contain a therapeutic ingredient for skin diseases other than the oxidized coenzyme Q and reduced coenzyme Q. As such ingredient, there may be mentioned those drugs which are generally used in the area of dermatological treatment, for example anti-inflammatory agents, immunosuppressives, antibacterial substances, antifungal agents, and disinfectants and, further, such antioxidant substances or skin activating ingredients as mentioned above.

When the therapeutic composition for skin diseases according to the invention is intended for use in the treatment of atopic dermatitis, it preferably further contains a therapeutic agent for atopic dermatitis other than the oxidized coenzyme Q and reduced coenzyme Q. Such therapeutic agent for atopic dermatitis may be any of those generally used in the treatment of atopic dermatitis, including steroids, more specifically prednisolone valerate acetate, amcinonide, diflucortolone valerate, dexamethasone valerate, clobetasol propionate, diflorasone diacetate, dexamethasone propionate, betamethasone dipropionate, difluprednate, fluocinonide, halcinonido, budesonide, hydrocortisone butyrate propionate, betamethasone valerate, beclomethasone dipropionate, fluocinolone acetonide, triamcinolone acetonide, flumethasone pivalate, hydrocortisone butyrate, clobetasone butyrate, alclometasone dipropionate, dexamethasone, methylprednisolone acetate, prednisolone, and hydrocortisone acetate, and other drugs than steroids, for example tacrolimus, and antihistamines.

The dermal composition of the present invention can be used as a cosmetic composition or a skin health care composition. Specific uses include, but are not limited to, cleansers, eye creams, eyeshadows, creams, milky lotions, skin lotions, perfumes, face powders, cosmetic oils, paste perfumes, powders, packs, shaving creams, shaving lotions, suntan oils, anti-suntan oils, suntan lotions, anti-suntan lotions, nail creams, nail enamels, bath cosmetics, rouge, mascara, lipsticks, lip creams, eyeliners, deodorants, cologne waters, etc.

In this case, the above composition may contain one or more of those cosmetic auxiliaries so far used in the conventional cosmetic or skin health care compositions, for example preservatives, bactericides, perfumes, antifoaming agents, colorants, coloring pigments, thickeners, surfactants, emulsifiers, softening agents, moistening agents and/or humectants, fats, oils, waxes and, further, alcohols, polyols, polymers, foam stabilizers, electrolytes, organic solvents, silicone derivatives, and other ingredients.

The dermal composition of the present invention can be used also as a bath salt or like composition. The bath salt or like composition so referred to herein means a composition to be dissolved in cold or warm water for use thereof at the time of bathing. The bath salt or like composition of the present invention may comprise additive and other ingredients conventionally used in bath salt preparations.

### BRIEF DESCRIPTION OF THE DRAWING

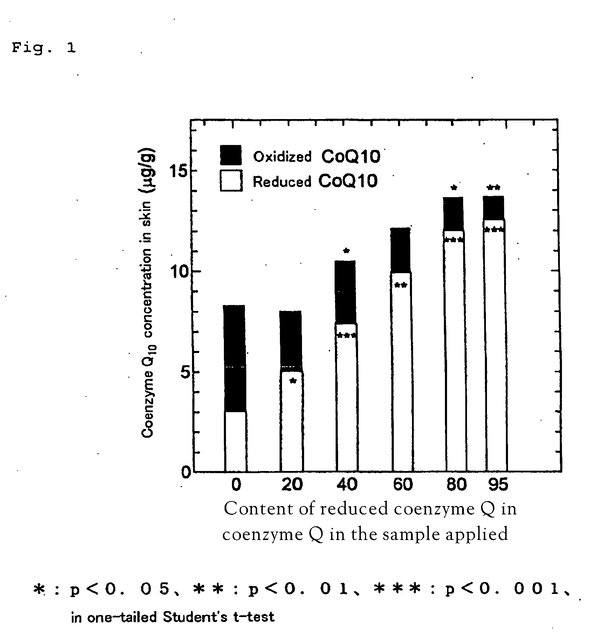

Fig. 1 is a graphic representation of the relationship between the concentration of coenzyme Q₁₀ in skin and the content of reduced coenzyme Q₁₀ in sample. The vertical axis denotes the total concentration of coenzyme Q₁₀ in skin, and the horizontal axis denotes the content of reduced coenzyme Q₁₀ in coenzyme Q₁₀ in the sample applied. Each bar represents the mean standard deviation (n = 4 or 5).

### BEST MODES FOR CARRYING OUT THE INVENTION

The following examples and preparation examples illustrate the present invention in more detail.

### (Example 1)

### (1) Preparation of test sample 1

Reduced coenzyme Q₁₀ (0.1 g; containing about 5% of oxidized coenzyme Q₁₀) was melted on a water bath at 50°C. Thereto was added polyethylene glycol 1500 (PEG 1500) melted in the same manner to make a total amount of 10 ml. This was made homogeneous by melting and mixing at 50°C and then allowed to solidify at room temperature to give an ointment-like composition.

### (2) Preparation of comparative sample 1

Oxidized coenzyme Q₁₀ (0.1 g) was melted on a water bath at 50°C. Thereto was added PEG 1500 to make a total amount of 10 ml. This was made homogeneous by melting and mixing at 50°C and then allowed to solidify at room temperature to give an ointment-like composition.

### (3) Percutaneous absorption test

The test sample 1 and comparative sample 1 were used as test substances. The test was carried out using male hairless rats (weighing 250 to 300 g) fed under well-fed conditions. A 0.1-g portion of the test sample 1, comparative sample 1, or PEG 1500 as a control was applied to an area of 3 cm square on the back of each hairless rat lightly anesthetized with ether. Three hours, 8 hours or 24 hours after application, the rat was sacrificed by euthanasia, the applied area was washed thoroughly, and a skin sample was taken. The skin sample was homogenized and extracted with propanol, the extract was concentrated using a solid phase column, and the amount of coenzyme Q₁₀ in the skin was determined by high-performance liquid chromatography. The total amount of coenzyme Q₁₀ in each skin sample is shown in Table 1. The numerical value shows the mean value ± standard deviation.

**Table 1**

| | Coenzyme Q₁₀ concentration in skin µg/g) | | |
|---|---|---|---|
| | 3hr | 8hr | 24hr |
| Control (PEG 1500) | 1.51 ± 0.38 | 1.35 ± 0.39 | 1.62 ± 0.50 |
| Oxidized coenzyme Q₁₀ | 8.32 ± 1.35 | 7.87 ± 1.75 | 7.63 ± 2.69 |
| | (100) | (100) | (100) |
| Reduced coenzyme Q₁₀^{#1} | 13.72 ± 0.70 | 17.96 ± 4.85 | 15.68± 3.95 |
| | (165***) | (228*) | (206*) |

| | | | |
|---|---|---|---|
| Mean ± SD, n = 3 to 8. *: p < 0.05, ***: p < 0.001, in one-tailed Student's t-test. #1: Containing about 5% of oxidized coenzyme Q₁₀. | | | |

As shown above, it was revealed that the coenzyme Q₁₀ containing 95% of reduced coenzyme Q₁₀ is very effective in increasing the amount of coenzyme Q₁₀ in skin as compared with 100% oxidized coenzyme Q₁₀.

Each of the skin samples mentioned above was homogenized and extracted with hexane, the extract was evaporated to dryness and dissolved in ethanol, and the proportion of reduced coenzyme Q₁₀ in skin was determined by high-performance liquid chromatography with an electrochemical detector. The amounts of reduced coenzyme Q₁₀ in skin thus found are shown in Table 2. Each numerical value means the mean ± standard deviation.

**Table 2**

| | Reduced coenzyme Q₁₀ concentration in skin (µg/g) | | |
|---|---|---|---|
| | 3hr | 8hr | 24hr |
| Control (PEG 1500) | 1.11 ± 0.26 | 0.84 ± 0.23 | 1.13 ± 0.39 |
| Oxidized coenzyme Q₁₀ | 3.02 ± 1.22 | 4.99 ± 2.12 | 5.44 ± 2.36 |
| | (100) | (100) | (100) |
| Reduced coenzyme Q₁₀^{#1} | 12.55 ± 0.51 | 15.84 ± 4.56 | 12.99 ± 4.81 |
| | (414***) | (317 **) | (239*) |

| | | | |
|---|---|---|---|
| Mean ± SD, n = 3 to 8. *: p < 0.05, **: p < 0.01, ***: p < 0.001, in one-tailed Student's t-test. #1: Containing about 5% of oxidized coenzyme Q₁₀. | | | |

As shown above, it was revealed that the coenzyme Q₁₀ containing 95% of reduced coenzyme Q₁₀ is very effective in increasing the amount of reduced coenzyme Q₁₀ in skin as compared with 100% oxidized coenzyme Q₁₀. Although the amount of reduced coenzyme Q₁₀ in skin is gradually increased by the reduction of oxidized coenzyme Q₁₀ in the treated skin, the rate thereof is not very rapid. Even after 24 hours after application of oxidized coenzyme Q₁₀, the amount of reduced coenzyme Q₁₀ in skin is only half or less as compared with the level 3 hours after application of reduced coenzyme Q₁₀.

### (Example 2)

### (1) Preparation of test sample 2

The sample was prepared in the same manner as described above in Example 1 for test sample 1 except that a mixture of oxidized coenzyme Q₁₀ and reduced coenzyme Q₁₀ in a mixing ratio of 80:20 by weight was used.

### (2) Preparation of test sample 3

The sample was prepared in the same manner as described above in Example 1 for test sample 1 except that a mixture of oxidized coenzyme Q₁₀ and reduced coenzyme Q₁₀ in a mixing ratio of 60:40 by weight was used.

### (3) Preparation of test sample 4

The sample was prepared in the same manner as described above in Example 1 for test sample 1 except that a mixture of oxidized coenzyme Q₁₀ and reduced coenzyme Q₁₀ in a mixing ratio of 40:60 by weight was used.

### (4) Preparation of test sample 5

The sample was prepared in the same manner as described above in Example 1 for test sample 1 except that a mixture of oxidized coenzyme Q₁₀ and reduced coenzyme Q₁₀ in a mixing ratio of 20:80 by weight was used.

### (5) Percutaneous absorption test

The test was carried out in the same manner as in Example 1 using the test samples 2, 3, 4 and 5 as well as the comparative sample 1 as test samples.

The results of the test are shown in Fig. 1. In Fig. 1, the vertical axis denotes the total amount of coenzyme Q₁₀ and the amount of reduced coenzyme Q₁₀ in skin at 3 hours after application, and the horizontal axis denotes the content (% by weight) of reduced coenzyme Q₁₀ relative to the total amount of coenzyme Q₁₀ in the sample applied. Each bar indicates the mean value.

As is evident from Fig. 1, the composition in which the proportion of reduced coenzyme Q₁₀ was 20% by weight gave a significantly increased concentration of reduced coenzyme Q₁₀ in skin as compared with the composition comprising oxidized coenzyme Q₁₀ alone. Further, with the composition containing reduced coenzyme R₁₀ in a proportion of 40% by weight, a still higher concentration was observed as compared with the composition containing reduced coenzyme Q₁₀ in a proportion of 20% by weight. From these results, it was revealed that when it contains not less than 20% by weight of reduced coenzyme Q₁₀, the composition of the present invention can undoubtedly increase the amount of reduced coenzyme Q₁₀ in skin as compared with the composition containing oxidized coenzyme Q₁₀ alone or the composition containing less than 20% by weight of reduced coenzyme Q₁₀ relative to the total amount of coenzyme Q₁₀.

### (Example 3)

### Therapeutic effect in atopic dermatitis model mice (NC mice) - 1

The method of Hirasawa et al. (Oyo Yakuri (Applied Pharmacology), Vol. 59, No. 6, pp. 123-134, 2000) was used for the evaluation. Ointments containing oxidized coenzyme Q₁₀ and ointments containing reduced coenzyme Q₁₀ (containing 5% of oxidized coenzyme Q₁₀ in coenzyme Q₁₀) were evaluated for therapeutic effect in atopic dermatitis model mice (NC mice). Dermatitis was induced in each group of 7 NC mice by sensitizing (once a week) using a hapten. On the occasion of the third sensitization, the treatment with each test compound was started. The coenzyme Q₁₀-containing ointment (1%) was applied at a dose of 0.1 g every day, while the positive control prednisolone ointment was applied once every other day. In the group in which the prednisolone ointment and the coenzyme Q₁₀ ointment were used combinedly, the ointments were applied alternately. The therapeutic effect was evaluated on a scoring scale of 0 to 3 (0: no symptom, 1: slight, 2: medium, 3: severe) for the 5 items: 1 - pruritus, 2 - rubefaction, bleeding, 3 - edema, 4 - abrasion, tissue deficit, 5 -crusting, dryness. The differences between the dermatitis scores at the start of the test and those on day 15 after the start of application are shown in Table 3. Each data indicates the mean ± standard deviation.

**Table 3**

| Test group | Increase in dermatitis score |
|---|---|
| Control group | 4.4 ± 1.18 (100) |
| 1% Oxidized coenzyme Q₁₀ ointment | 3.3 ± 2.14 (75) |
| 1% Reduced coenzyme Q₁₀ ointment* | 3.1 ± 2.04 (70) |
| Prednisolone ointment (P) | 2.1 ± 1.35 (48) |
| Prednisolone ointment (P) | 2.1 ± 1.35 (100) |
| P + 1 % oxidized coenzyme Q₁₀ ointment | 1.1 ± 1.07 (52) |
| P + 1% reduced coenzyme Q₁₀ointment* | -1.3 ± 1.98 (-) |

| | |
|---|---|
| Mean ± SD, n = 7 * Total coenzyme Q₁₀ contained about 5% of oxidized coenzyme Q₁₀. | |

A greater score value indicates a higher level of aggravation of dermatitis during testing. In the oxidized, and reduced coenzyme Q₁₀ ointment groups, the ointments showed an obvious aggravation preventing effect, like in the positive control prednisolone ointment, as compared with the control group. In the group of combined use with prednisolone, a more powerful therapeutic effect was shown as compared with the group of single use of prednisolone, and the reduced coenzyme Q₁₀ ointment, in particular, gave a score lower than the score at the start of testing, indicating its dermatitis healing ability. It has so far been quite unknown in the art that ointments containing a coenzyme Q as its main active ingredient is actually effective against atopic dermatitis in the manner mentioned above. Furthermore, it has never been anticipated that when used combinedly with a steroid, a coenzyme Q can show such a more potent effect.

### (Example 4)

### Therapeutic effect in atopic dermatitis model mice (NC mice) - 2

The effect of the single use of a high concentration coenzyme Q₁₀ ointment (10%) and the effect of the combined use of Protopic ointment (tacrolimus preparation), a therapeutic agent for atopic dermatitis, and a low concentration coenzyme Q₁₀ ointment (1%) were examined by carrying out the same test as in Example 3. In the single use evaluation group, the test ointment was applied every day and, in the combined use evaluation group, Protopic ointment was applied at a does of 0.1 g once a week and 0.1 g of the low concentration coenzyme Q₁₀ ointment on the remaining 6 days per week. In a control group, Protopic ointment was applied singly 6 times a week. In a positive control group, a prednisolone ointment was applied every other day. The results obtained on the 15th day after commencement of application are shown in Table 4. Each value indicates the mean ± standard deviation.

**Table 4**

| Test group | Increase in dermatitis score |
|---|---|
| Control group | 4.1 ± 0.90 (100) |
| 10% Oxidized coenzyme Q₁₀ ointment | 4.0 ± 1.53 (98) |
| 10% Reduced coenzyme Q₁₀ ointment* | 2.9 ± 1.21 (71) |
| Prednisolone ointment (P) | 2.7 ± 2.14 (66) |
| Protopic ointment (P) | 5.4 ± 1.90 (100) |
| P + 1% oxidized coenzyme Q₁₀ ointment | 3.7 ± 1.80 (69) |
| P + 1 % reduced coenzyme Q₁₀ ointment* | 3.0 ± 1.73 (56) |

| | |
|---|---|
| Mean ± SD, n = 7 * Total coenzyme R₁₀ contained about 5% of oxidized coenzyme Q₁₀. | |

The high-concentration reduced coenzyme Q₁₀ ointment was roughly comparable in therapeutic effect to the positive control prednisolone ointment, indicating that it can show a potent therapeutic effect even when used singly. On the other hand, Protopic ointment in the single use group showed no efficacy probably due to the small number of applications. However, when Protopic ointment was used in combination with the low concentration coenzyme Q₁₀ ointment, a distinct synergistic effect was shown and aggravation was suppressed. That the coenzyme Q₁₀ ointments used combinedly with tacrolimus also showed a synergistic effect like in the combined use with the steroid preparation indicates that the synergistic effect of the coenzyme Q₁₀ ointment on atopic dermatitis is not specific to the steroid preparation.

### (Example 5)

### Incised wound healing test in rats

SD rats (male, 12-week-old) were clipped of hairs and divided into groups of 10 animals to make the mean body weights of the groups roughly the same, and subjected to the test. Each animal was given an incision wound along the median line under diethyl ether anesthesia. The incision wound was stapled at three sites using Michel's clips, and a 1% oxidized coenzyme Q₁₀ ointment or a 1% reduced coenzyme Q₁₀ ointment was applied at a dose of 0.2 g/day for 4 days. Two control groups, namely a nontreated group and an ointment base group treated with the same dose of the ointment base, were used. Three days after incision, the Michel's clips were removed and, four days after incision, each animal was euthanized by overanesthesia with diethyl ether, the skin around the incision was peeled off, and skin sections were prepared. The skin sections were measured for tension on a tensile tester.

As a result, it was noted that oxidized coenzyme Q₁₀ and reduced coenzyme Q₁₀ have a skin repair promoting effect.

### (Example 6)

### Oxidation stability evaluation of reduced coenzyme Q₁₀ in ointment

Reduced coenzyme Q₁₀-containing ointments were evaluated for oxidation stability. The ointment bases used were PEG 1500, a hydrophilic ointment, an absorptive ointment, and a simple ointment. The PEG 1500 used was a product of Wako Pure Chemical Industries, and the hydrophilic ointment, absorptive ointment and simple ointment used were respectively the products according to the Japanese Pharmacopoeia. Using the respective bases and reduced coenzyme Q₁₀, ointments were prepared in the same manner as in Example 1. The thus-prepared reduced coenzyme Q₁₀ ointments were stored at 23°C for 2 weeks either in air or in a vessel purged with nitrogen, and the proportion of the reduced form of coenzyme Q₁₀ in each ointment was determined by HPLC. The results thus obtained are shown in Table 5.

**Table 5**

| Base | Concentration (%)^{*1} | Proportion of reduced coenzyme Q₁₀ (%)^{*2} | | |
|---|---|---|---|---|
| | | 4° C in air | 23° C in air | 23° C in nitrogen |
| PEG1500 | 1 | 87.5 | 56.4 | 62.3 |
| PEG1500 | 10 | 92.5 | 94.4 | 93.6 |
| Hydrophilic ointment | 1 | - | 75.1 | 79.3 |
| Absorptive ointment | 1 | - | 29.8 | 5.3 |
| Simple ointment | 1 | - | 83.9 | 83.6 |

| | | | | |
|---|---|---|---|---|
| Mean, n = 2 *1) Concentration of coenzyme Q₁₀ in ointment *2) Proportion of reduced coenzyme Q₁₀ in total coenzyme R₁₀ in ointment after 2 weeks of storage under respective conditions. - Not tested. | | | | |

In the reduced coenzyme Q₁₀ ointments prepared by using simple ointment and hydrophilic ointment, respectively, as bases, about 80% of coenzyme Q₁₀ retained the reduced form after the 2 weeks of storage whereas, in the PEG 1500-based and absorptive ointment-based ointments, only 60% and 30%, respectively, of the reduced form remained. As regards the oxidation stability of reduced coenzyme Q₁₀ in the ointments, the substitution of the storage vessel atmosphere with nitrogen showed no protective effect. When the PEG 1500-based ointment was stored at 4°C in a refrigerator, the enzyme stability was assured for 2 weeks. Evaluation of the dependency on the concentration of reduced coenzyme Q₁₀ in ointment revealed that the 10% ointment is higher in stability than the 1% preparation, namely the higher the concentration is, the more stable the preparation is.

### (Preparation Example 1)

A coenzyme Q₁₀-containing hydrophilic ointment was prepared by a conventional method according to the following formulation.

| | |
|---|---|
| Hydrophilic ointment | 99.000% by weight |
| coenzyme Q₁₀ | 1.000% by weight |

### (Preparation Example 2)

A coenzyme Q₁₀-containing W/O cream was prepared by a conventional method according to the following formulation.

| | | |
|---|---|---|
| Glycerol sorbitan fatty acid ester | | 6.000 % by weight |
| Microcrystalline wax | | 1.000% by weight |
| Olive oil | | 3.000% by weight |
| Liquid paraffin | | 19.000% by weight |
| Magnesium stearate | | 1.000% by weight |
| Propylene glycol | | 3.700% by weight |
| Magnesium sulfate (MgSO₄·7H₂O) | | 0.700% by weight |
| Coenzyme Q₁₀ | | 1.000% by weight |
| Dehydrated salt | to make | 100.000% by weight |

### (Preparation Example 3)

A coenzyme Q₁₀-containing W/O emulsion was prepared by a conventional method according to the following formulation.

| | | |
|---|---|---|
| Polyoxyethylene glycerol sorbitan fatty acid ester | | |
| | | 3.600% by weight |
| Polyoxyethylene fatty acid ester | | 1.400% by weight |
| Cetearyl alcohol | | 2.000% by weight |
| Mineral oil, GP 9 | | 20.000% by weight |
| Paraben mixture | | q.v. |
| Magnesium sulfate (MgSO₄·7H₂O) | | 0.700% by weight |
| Coenzyme Q₁₀ | | 1.000% by weight |
| Calcium chloride (CaCl₂) | | 0.85% by weight |
| Dehydrated salt | to make | 100.000% by weight |

### (Preparation Example 4)

A coenzyme Q₁₀-containing W/O lotion was prepared by a conventional method according to the following formulation.

| | | |
|---|---|---|
| Glycerol sorbitan fatty acid ester | | 1.300% by weight |
| Polyoxyethylene fatty acid ester | | 3.700% by weight |
| Neutral oil | | 6.000% by weight |
| Liquid paraffin, GP 9 | | 14.000% by weight |
| Propylene glycol | | 3.800% by weight |
| Magnesium sulfate (MgSO₄·7H₂O) | | 0.700% by weight |
| Ribonic acid | | 1.500% by weight |
| Coenzyme Q₁₀ | | 1.000% by weight |
| Desalted water | to make | 100.000% by weight |

### INDUSTRIAL APPLICABILITY

The composition of the present invention, which has the above constitution, is excellent in percutaneous absorption of coenzyme Q₁₀ and highly effective in the treatment of skin diseases, such as atopic dermatitis, and in skin health care.

## Claims

1. A composition for dermal application, which comprises, as an active ingredient, both an oxidized coenzyme Q represented by the formula (1): 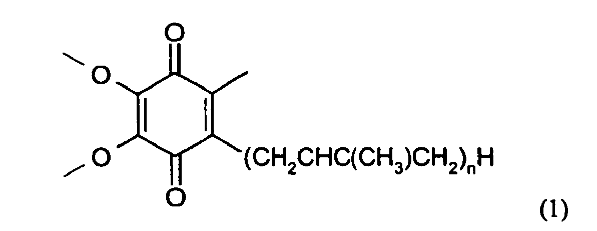 in which n represents an integer of 1 to 12, and a reduced coenzyme Q represented by the formula (2) : 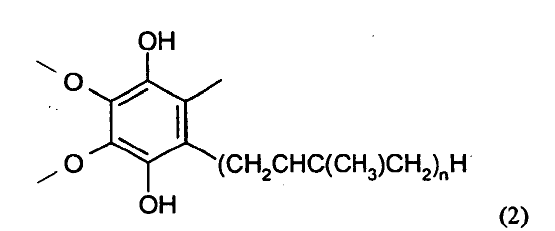 in which n represents an integer of 1 to 12, wherein
the total content of the oxidized coenzyme Q and the reduced coenzyme Q is 0.01 to 99 % by weight relative to the whole amount of the composition, and the proportion of the reduced coenzyme Q relative to the total amount of the oxidized coenzyme Q represented by the formula (1) and the reduced coenzyme Q represented by the formula (2) is not less than 20 % by weight.

2. The composition for dermal application according to Claim 1, wherein the proportion of the reduced coenzyme Q relative to the total amount of the oxidized coenzyme Q represented by the formula (1) and the reduced coenzyme Q represented by the formula (2) is not less than 40 % by weight.

3. The composition for dermal application according to Claim 1 or 2, wherein the proportion of the reduced coenzyme Q relative to the total amount of the oxidized coenzyme Q represented by the formula (1) and the reduced coenzyme Q represented by the formula (2) is not more than 95 % by weight.

4. The composition for dermal application according to any of Claims 1 to 3, wherein the oxidized coenzyme Q represented by the general formula (1) is oxidized coenzyme Q₁₀ and the reduced coenzyme Q represented by the general formula (2) is reduced coenzyme Q₁₀.

5. The composition for dermal application according to any of Claims 1 to 4, which is to be applied to a human.

6. The composition for dermal application according to any of Claims 1 to 4, which is to be applied to pets, a domestic animal and/or a bird.

7. The composition for dermal application according to Claim 6, which is to be applied to a dog and/or a cat.

8. A therapeutic composition for skin diseases which comprises the composition for dermal application according to any of Claims 1 to 7.

9. The therapeutic composition for skin diseases according to Claim 8, which further comprises a therapeutic ingredient for skin diseases other than the oxidized coenzyme Q represented by the formula (1) and other than the reduced coenzyme Q represented by the formula (2).

## Patentansprüche

1. Eine Zusammensetzung für die dermatologische Anwendung, welche umfasst, als einen aktiven Bestandteil, sowohl ein oxidiertes Coenzym Q, welches durch die Formel (1) dargestellt wird: 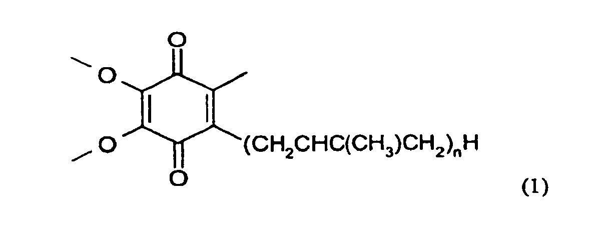 in welcher n einer ganzzahligen Zahl von 1 bis 12 entspricht, und ein reduziertes Coenzym Q, welches durch die Formel (2) dargestellt wird: 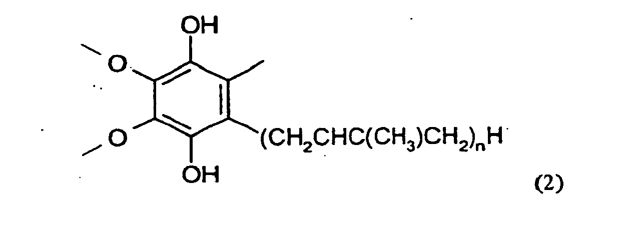 in welcher n einer ganzzahligen Zahl von 1 bis 12 entspricht, worin die Gesamtmenge an dem oxidierten Coenzym Q und an dem reduzierten Coenzym Q 0,01 bis 99 Gew.-%, bezogen auf die gesamte Menge der Zusammensetzung, beträgt und das Verhältnis des reduzierten Coenzyms Q relativ zu der gesamten Menge des oxidierten Coenzyms Q, welches durch die Formel (1) dargestellt wird, und wobei das reduzierte Coenzym Q durch die Formel (2) dargestellt wird, nicht weniger als 20 Gew.-% beträgt.

2. Die Zusammensetzung für die dermatologische Anwendung gemäß Anspruch 1 , worin das Verhältnis des reduzierten Coenzyms Q, bezogen auf die gesamte Menge des oxidierten Coenzyms Q, welches durch die Formel (1) dargestellt wird, und wobei das reduzierte Coenzym Q durch die Formel (2) dargestellt wird, nicht weniger als 40 Gew.-% beträgt.

3. Die Zusammensetzung für die dermatologische Anwendung gemäß Anspruch 1 oder 2, worin das Verhältnis des reduzierten Coenzyms Q, bezogen auf die gesamte Menge des oxidierten Coenzyms Q, welches durch die Formel (1) dargestellt wird, und wobei das reduziert Coenzym Q durch die Formel (2) dargestellt wird, nicht weniger als 95 Gew.-% beträgt.

4. Die Zusammensetzung für die dermatologische Anwendung gemäß einem der Ansprüche 1 bis 3, worin das oxidierte Coenzym Q, welches durch die allgemeine Formel (1) dargestellt wird, oxidiertes Coenzym Q₁₀ ist und das reduzierte Coenzym Q, welches durch die allgemeine Formel (2) dargestellt wird, reduziertes Coenzym Q₁₀ ist.

5. Die Zusammensetzung für die dermatologische Anwendung gemäß einem der Ansprüche 1 bis 4, welche an einem Menschen angewendet wird.

6. Die Zusammensetzung für die dermatologische Anwendung gemäß einem der Ansprüche 1 bis 4, welche an Tieren, an einem Haustier und/oder an einem Vogel angewendet wird.

7. Die Zusammensetzung für die dermatologische Anwendung gemäß Anspruch 6, welche angewendet wird an einem Hund und/oder an einer Katze.

8. Eine therapeutische Zusammensetzung für Hautkrankheiten, welche die Zusammensetzung für die dermatologische Anwendung gemäß einem der Ansprüche 1 bis 7 umfasst.

9. Die therapeutische Zusammensetzung für Hautkrankheiten gemäß Anspruch 8, welche darüber hinaus einen therapeutischen Bestandteil für Hautkrankheiten umfasst, welcher anders ist als das oxidierte Coenzym Q, welches durch die Formel (1) dargestellt wird, und anders ist als das reduzierte Coenzym Q, welches durch die Formel (2) dargestellt wird.

## Revendications

1. Composition pour application dermique, qui comprend comme ingrédient actif, à la fois une co-enzyme Q oxydée représentée par la formule (1) : 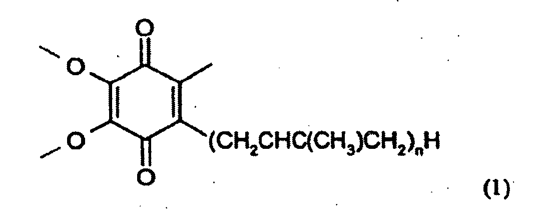 dans laquelle n représente un nombre entier de 1 à 12, et une co-enzyme Q réduite représentée par la formule (2) : 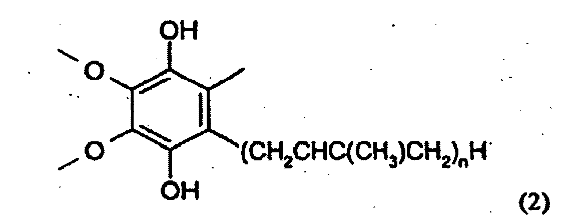 dans laquelle n représente un nombre entier de 1 à 12, dans laquelle
la teneur totale en co-enzyme Q oxydée et en co-enzyme Q réduite est de 0,01 à 99 % en poids par rapport à la quantité totale de la composition, et la proportion de co-enzyme Q réduite par rapport à la quantité totale de co-enzyme Q oxydée représentée par la formule (1) et de co-enzyme Q réduite représentée par la formule (2) n'est pas inférieure à 20 % en poids.

2. Composition pour application dermique selon la revendication 1, dans laquelle la proportion de co-enzyme Q réduite par rapport à la quantité totale de co-enzyme Q oxydée représentée par la formule (1) et de co-enzyme Q réduite représentée par la formule (2) n'est pas inférieure à 40 % en poids.

3. Composition pour application dermique selon la revendication 1 ou 2, dans laquelle la proportion de co-enzyme Q réduite par rapport à la quantité totale de co-enzyme Q oxydée représentée par la formule (1) et de co-enzyme Q réduite représentée par la formule (2) n'est pas supérieure à 95 % en poids.

4. Composition pour application dermique selon l'une quelconque des revendications 1 à 3, dans laquelle la co-enzyme Q oxydée représentée par la formule générale (1) est la co-enzyme oxydée Q₁₀ et la co-enzyme Q réduite représentée par la formule générale (2) est la co-enzyme réduite Q₁₀.

5. Composition pour application dermique selon l'une quelconque des revendications 1 à 4, qui est destinée à être appliquée sur un être humain.

6. Composition pour application dermique selon l'une quelconque des revendications 1 à 4, qui est destinée à être appliquée sur des animaux de compagnie, un animal domestique et/ou un oiseau.

7. Composition pour application dermique selon la revendication 6, qui est destinée à être appliquée sur un chien et/ou un chat.

8. Composition thérapeutique pour les maladies de la peau, qui comprend la composition pour application dermique selon l'une quelconque des revendications 1 à 7.

9. Composition thérapeutique pour les maladies de la peau selon la revendication 8, qui comprend en outre un ingrédient thérapeutique pour les maladies de la peau, autre que la co-enzyme Q oxydée représentée par la formule (1) et autre que la co-enzyme Q réduite représentée par la formule (2).
